# EUROPÄISCHE PATENTSCHRIFT

(11) **EP 0 359 893 B1**
(45) Veröffentlichungstag und Bekanntmachung des Hinweises auf die Patenterteilung: **18.11.1993**
(21) Anmeldenummer: 88890238.4
(22) Anmeldetag: 21.09.1988
(51) Int. Cl.: A61N 1/16, H05K 9/00

(54) **Einrichtung zur Abschirmung gegen ein elektromagnetisches Feld**
Device for shielding against electromagnetic fields
Dispositif de protection contre des champs électromagnétiques

(43) Veröffentlichungstag der Anmeldung: 28.03.1990
(73) Patentinhaber: Andrae, Franz, A-1210 Wien (AT)
(72) Erfinder: Andrae, Franz, A-1210 Wien (AT)
(74) Vertreter: Holzer, Walter, Dipl.-Ing.

(56) Entgegenhaltungen:
- EP-A- 0 277 819
- BE-A- 341 575
- DE-U- 8 701 667
- GB-A- 766 886

## Beschreibung

Die Erfindung betrifft eine Einrichtung zur Abschirmung gegen ein elektromagnestisches Feld, bestehend aus einer Abschirmfolie aus Metall, insbesondere Aluminium, oder metallisiertem Kunststoff, die mit Durchbrechungen versehen ist.

Eine aus der DE-OS 32 32 224 bekannte Einrichtung dieser Art hat Rechteckform, wobei die Folie auf ihrer gesamten Fläche oder auf Teilen dieser Fläche gleichmäßig oder ungleichmäßig verteilte Ausnehmungen aufweist und auf einem Trägermaterial aufkaschiert wird. Die Herstellung einer solchen Folie und ihre Anwendung sind jedoch relativ aufwendig. Außerdem ergibt in der Praxis die Rechteckform nur eine beschränkte Abschirmwirkung.

Die Erfindung zielt darauf ab, eine leicht zu handhabende, den jeweiligen Erfordernissen anpaßbare Einrichtung der einleitend angegebenen Art zu schaffen, und erreicht dies erfindungsgemäß dadurch, daß die Abschirmfolie im wesentlichen sechseckigen Umriß hat und durch die Ecken mit der Folienmitte verbindende Stege in sechs Dreiecksfelder unterteilt ist, und daß die Durchbrechungen durch zur Sechseckseite jedes Dreiecksfeldes und zueinander parallele Schlitze gebildet sind, die sich über das gesamte Dreiecksfeld erstrecken.

Die erfindungsgemäße Einrichtung hat den Vorteil, daß sie auf einfache Weise als Einlage in einem widerstandsfähigen Körper, vorzugsweise einem Holzkörper, verwendet werden kann, der leicht zu handhaben ist und dessen Form im Bedarfsfall die Aneinanderreihung mehrerer Körper ermöglicht. Die Einrichtung hat sich überraschenderweise als äußerst wirksam gegen die schädliche Wirkung von elektromagnetischen Feldern erwiesen, wobei sie offenbar wegen ihrer symmetrischen Form für alle Feldrichtungen zumindest angenähert parallele Leitungsabschnitte darbietet. Die Einrichtung kann auf einfache Weise z.B. im Bereich eines Bettes, einer Sitzstelle od.dgl. angeordnet werden.

Gemäß einer bevorzugten Ausführungsform der Erfindung ist die Abschirmfolie zwischen zwei im wesentlichen sechseckigen Platten aus Sperrholz od.dgl. angeordnet, die miteinander verleimt sind. Dabei kann in einer der Platten eine kreisförmige Ausnehmung zur Aufnahme der Abschirmfolie ausgebildet sein.

Die Erfindung wird nachfolgend an einem Ausführungsbeispiel unter Bezugnahme auf die Zeichnung näher erläutert, in der Fig. 1 eine Draufsicht auf eine Einrichtung gemäß der Erfindung zeigt, und die Fig. 2 und 3 eine Draufsicht und einen Schnitt nach der Linie 3 - 3 einer zur Aufnahme der erfindungsgemäßen Einrichtung dienenden Platte.

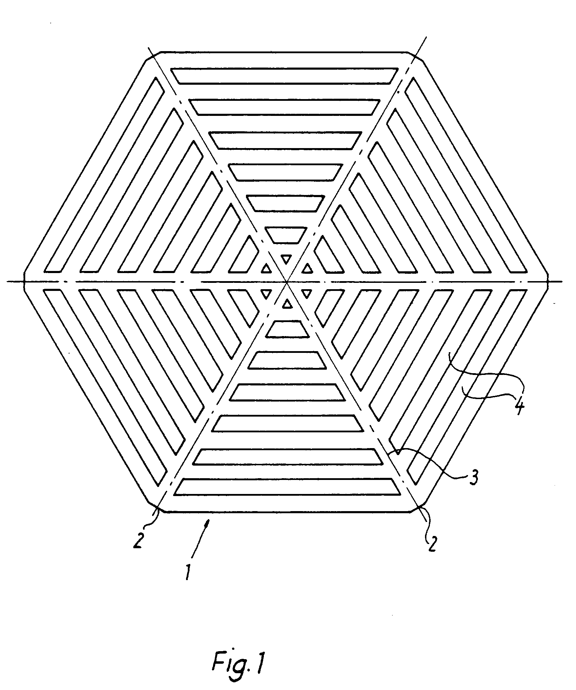

Gemäß Fig. 1 hat die aus Aluminium bestehende Abschirmfolie 1 im wesentlichen sechseckigen Umriß und ist durch die Ecken 2 mit der Folienmitte verbindende Stege 3 in sechs Dreiecksfelder unterteilt. Die Durchbrechungen 4 sind durch zur Sechseckseite jedes Dreiecksfeldes und zueinander parallele Schlitze gebildet, die sich über das gesamte Dreiecksfeld erstrecken.

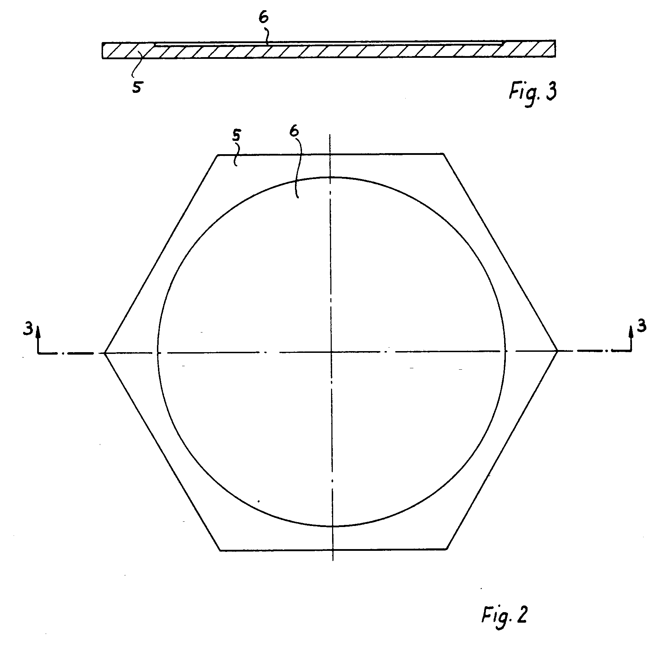

Die Abschirmfolie 1 wird zwischen zwei im wesentlichen sechseckigen Platten 5 aus Sperrholz od.dgl. angeordnet, die miteinander verleimt werden. In einer der Platten 6, die in den Fig. 2 und 3 gezeigt ist, ist eine kreisförmige Ausnehmung 6 zur Aufnahme der Abschirmfolie 1 ausgebildet. Der die Abschirmfolie aufnehmende Holzkörper könnte aber auch anderen Umriß als die Abschirmfolie haben.

## Patentansprüche

1. Einrichtung zur Abschirmung gegen ein elektromagnetisches Feld, bestehend aus einer Abschirmfolie aus Metall, insbesondere Aluminium, oder metallisiertem Kunststoff, die mit Durchbrechungen versehen ist, dadurch gekennzeichnet, daß die Abschirmfolie (1) im wesentlichen sechseckigen Umriß hat und durch die Ecken (2) mit der Folienmitte verbindende Stege (3) in sechs Dreiecksfelder unterteilt ist, und daß die Durchbrechungen (4) durch zur Sechseckseite jedes Dreiecksfeldes und zueinander parallele Schlitze gebildet sind, die sich über das gesamte Dreiecksfeld erstrecken.

2. Einrichtung nach Anspruch 1, dadurch gekennzeichnet, daß die Abschirmfolie (1) zwischen zwei im wesentlichen sechseckigen Platten (5) aus Sperrholz od.dgl. angeordnet ist, die miteinander verleimt sind.

3. Einrichtung nach Anspruch 2, dadurch gekennzeichnet, daß in einer der Platten (5) eine kreisförmige Ausnehmung (6) zur Aufnahme der Abschirmfolie (1) ausgebildet ist.

## Claims

1. Device for shielding against an electromagnetic field, consisting of a shielding foil made of metal, in particular aluminium, or metallised plastic, which is provided with through-holes, characterised in that the shielding foil (1) has an essentially hexagonal contour and is divided into six triangular fields by webs (3) connecting the corners (2) to the foil centre, and in that the through-holes (4) are formed by slots which are parallel to the hexagonal side of each triangular field and to one another, said slots extending over the entire triangular field.

2. Device according to Claim 1, characterised in that the shielding foil (1) is arranged between two essentially hexagonal panels (5) consisting of plywood or the like, which are bonded to each other.

3. Device according to Claim 2, characterised in that a circular recess (6) for receiving the shielding foil (1) is made in one of the panels (5).

## Revendications

1. Dispositif de protection contre un champ électromagnétique, constitué d'une feuille-écran en métal, en particulier en aluminium, ou en matière plastique métallisée, pourvue de jours (4), caractérisé en ce que la feuille-écran (1) a, en substance, un contour hexagonal et est subdivisée en six zones triangulaires par des plages (3) reliant les coins au centre de la feuille, et les jours (4) sont formés par des lentes parallèles à chaque côté de la zone triangulaire formant l'hexagone et parallèles entre elles, qui s'étendent sur l'ensemble de la zone triangulaire.

2. Dispositif suivant la revendication 1, caractérisé en ce que la feuille-écran (1) est placée entre deux plaques en substance hexagonales (5) en contreplaqué ou en matière similaire qui sont collées l'une à l'autre.

3. Dispositif suivant la revendication 2, caractérisé en ce que l'une des plaques (5) présente un évidement (6) de forme circulaire destiné à recevoir la feuille-écran (1).
